**Europäisches Patentamt**

**European Patent Office**

**Office européen des brevets**

(19)

(11) Veröffentlichungsnummer: **0 611 964 A2**

# (12) EUROPÄISCHE PATENTANMELDUNG

(21) Anmeldenummer: **94100354.3**

(22) Anmeldetag: **12.01.94**

(51) Int. Cl.5: **G01N 25/00**

(30) Priorität: **13.01.93 DE 4300495**

(43) Veröffentlichungstag der Anmeldung:
**24.08.94 Patentblatt 94/34**

(84) Benannte Vertragsstaaten:
**AT DE FR GB IT**

(71) Anmelder: **BEGO Bremer Goldschlägerei Wilh. Herbst GmbH & Co.**
**Emil-Sommer-Strasse 7-9**
**D-28329 Bremen (DE)**

(72) Erfinder: **Gundlach, Hans-Werner, Dr.**
**Beethovenstrasse 21**
**D-28209 Bremen (DE)**
Erfinder: **Weiss, Joachim**
**Massolleweg 4a**
**D-28355 Bremen (DE)**

(74) Vertreter: **Möller, Friedrich, Dipl.-Ing. et al**
**Meissner, Bolte & Partner**
**Anwaltssozietät**
**Hollerallee 73**
**D-28209 Bremen (DE)**

(54) **Verfahren zum Prüfen von Dentalgussmaterialien.**

(57) Dentalgußmaterialien werden üblicherweise aus Gold- und/oder Titanlegierungen gebildet. Wichtig ist dabei, daß der Anteil der einzelnen Legierungskomponenten den Anforderungen entspricht, weil die Legierungsbestandteile wesentlichen Einfluß auf die Eigenschaften des jeweiligen Dentalgußmaterials haben. Um die Dentalgußmaterialien auf die richtige Legierung zu überprüfen, ist es üblich, den zeitbezogenen Temperaturverlauf beim Schmelzen oder zeitbezogene temperaturproportionale Werte zu ermitteln. Diese allein lassen aber noch keine einfache und aussagekräftige Prüfung zu.

Erfindungsgemäß werden die ermittelten Zeit-/Temperaturwerte auf einen bestimmten Sollwert normiert und die normierten Zeit-/Temperaturwerte mit den Zeit-/Temperatursollwerten verglichen. Auf diese Weise ist eine einfache und präzise Prüfung möglich. Diese Prüfung kann einfachstenfalls visuell erfolgen.

Fig. 2

Die Erfindung betrifft ein Verfahren zum Prüfen von Dentalgußmaterialien gemäß dem Oberbegriff des Anspruchs 1.

Zur Herstellung von Dentalgußteilen werden üblicherweise aus mehreren Elementen zusammengesetzte Materialien (Legierungen) verwendet. Entscheidend für die Qualität der Dentalgußteile ist das Mengen-Verhältnis der einzelnen Elemente, insbesondere die Menge der Legierungsbestandteile. Deswegen müssen die Dentalgußmaterialien auf ihre richtige Legierung geprüft werden.

Es ist bekannt, daß der zeitliche Temperaturverlauf beim Schmelzen des Dentalgußmaterials Rückschlüsse auf die jeweilige Legierung zuläßt, weil jede Legierung einen charakteristischen Temperatur-Zeitverlauf aufweist. Deshalb erfolgt eine Überprüfung der Dentalgußmaterialien anhand des Temperatur-Zeitverlaufs beim Schmelzen. Dabei hat sich jedoch die Auswertung des ermittelten Temperatur-Zeitverlaufs der Schmelze als schwierig erwiesen, weil zum einen die Abweichungen unterschiedlicher Legierungen zum Teil nur sehr gering sind und zum anderen sich die Temperatur-Zeitverläufe in Abhängigkeit von der jeweiligen Menge des zu schmelzenden Dentalgußmaterials ändern.

Aufgabe der vorliegenden Erfindung ist es, die Prüfung von Dentalgußmaterialien anhand ihres Temperatur-Zeitverlaufs zu vereinfachen und zuverlässiger zu machen.

Zur Lösung dieser Aufgabe weist das erfindungsgemäße Verfahren die Merkmale des Anspruchs 1 auf. Durch die Normierung der Istwerte darstellenden ermittelten Zeit-Temperaturwerte, womit stets auch zeit-temperaturproportionale Werte gemeint sind, auf mindestens einen bestimmten Sollwert, insbesondere Zeit-Temperatursollwert (einschließlich zeit-solltemperaturproportionale Werte), werden Mengenabweichungen der zu schmelzenden Dentalgußmaterialien eliminiert. Somit stellen die normierten Zeit-Temperaturwerte mengenunabhängige Istwerte dar, die die Prüfung erheblich vereinfachen. Infolge des weiterhin erfindungsgemäß erfolgenden Vergleichs der normierten Zeit-Temperaturistwerte mit Sollwerten, insbesondere Zeittemperatursollwerten, lassen sich relativ kleine Legierungsabweichungen ermitteln, wodurch eine präzise Prüfung gewährleistet ist.

Bei einem bevorzugten Verfahren erfolgt die Normierung der ermittelten Zeit-Temperaturistwerte auf einen einzigen (bestimmten) Zeit-Temperatursollwert, und zwar insbesondere auf einen Soliduspunkt. Hierbei handelt es sich um denjenigen Punkt, bei dem das feste Dentalgußmaterial anfängt, sich zu verflüssigen. Dieser Punkt wird charakterisiert durch die Solidustemperatur oder eine solidusproportionale Temperatur und den dazugehörigen Zeitpunkt, also denjenigen Punkt, an dem das feste Dentalgußmaterial beginnt, sich zu verflüssigen. Hierbei handelt es sich um einen charakteristischen Punkt, der mit relativ großer Genauigkeit bei jeder zu schmelzenden Menge des Dentalgußmaterials und jeder Legierung feststellbar ist.

Vorzugsweise wird verfahrensmäßig so vorgegangen, daß der Soliduspunkt der zu prüfenden Legierung auf den Soliduspunkt einer vorgegebenen Soll-Legierung bezogen wird. Dazu werden die Verhältnisse der Zeiten und der Temperaturwerte oder der temperaturproportionalen Werte an den Soliduspunkten zueinander ins Verhältnis gesetzt. Die sich dadurch ergebenden Verhältnisfaktoren (für die Zeit und die Temperaturen bzw. temperaturproportionalen Werte) dienen dazu, die übrigen ermittelten Zeit-Temperaturwerte der zu prüfenden Legierung umzurechnen und dadurch zu normieren. Auf diese Weise entstehen für vergleichbare Legierungen unterschiedlicher Mengen übereinstimmende Zeit-Temperaturwerte.

Der Vergleich der zu prüfenden Legierung mit der Soll-Legierung erfolgt dadurch, daß Temperaturwerte oder temperaturproportionale Werte gleicher Zeiten miteinander verglichen werden. Liegen die Abweichungen der normierten Zeit-Temperaturwerte der zu prüfenden Legierung innerhalb eines festgelegten, zulässigen Grenzwertes gegenüber dem jeweiligen Zeit-Temperatursollwert der vorgebenen Legierung, entspricht die zu prüfende Legierung den Vorgaben, ist also in Ordnung. Fallen hingegen mindestens einige der normierten Zeit-Temperaturwerte der zu prüfenden Legierung aus den zulässigen Streubereich der vorgegebenen Legierung heraus, ist das ein Zeichen dafür, daß die zu prüfende Legierung den Anforderungen nicht entspricht.

Nach einem bevorzugten Verfahren erfolgt die Prüfung der Dentalgußlegierung anhand von Diagrammen. Es wird dazu ein Vergleichsdiagramm mit der vorgegebenen (idealen) Legierung mit einem aus den normierten Zeit-Temperaturwerten der zu prüfenden Legierung gebildeten Prüfdiagramm verglichen. Auf diese Weise ist eine visuelle Kontrolle möglich.

Zur Erleichterung der insbesondere visuellen Kontrolle ist nach einer Weiterbildung der Erfindung vorgesehen, das Vergleichsdiagramm aus zwei (oberen und unteren) Kurven zu bilden, die obere und untere Grenzwerte der Zeit-/Temperatursollwerte darstellen. Der Vergleich mit dem Prüfdiagramm erfolgt dann dergestalt, daß das Prüfdiagramm in der zwischen der oberen und unteren Kurve eingeschlossenen Fläche des Vergleichsdiagramms liegen muß, wenn die zu prüfende Legierung den Anforderungen entspricht.

Eine weitere Besonderheit der Erfindung besteht darin, daß zumindest das (normierte) Prüfdiagramm von einem der Schmelz- oder Gießvorrichtung zugeordneten Drucker ausgedruckt wird. Dadurch sind die Prüfdaten praktisch in einem Gußprotokoll dokumentiert. Gleichzeitig läßt sich das Prüfdiagramm problemlos dem Vergleichsdiagramm gegenüberstellen, indem dieses beispielsweise als Schablone über das Prüfdiagramm gelegt wird. Alternativ ist es denkbar, sowohl das (normierte) Prüfdiagramm als auch das dazugehörende Vergleichsdiagramm (gegebenenfalls mit oberen und unteren Grenzwertkurven) gemeinsam auszudrucken. Daraufhin kann der Vergleich jederzeit problemlos vorgenommen werden. Er ist jederzeit nachvollziehbar, ohne daß dazu ein (separates) Vergleichsdiagramm vorhanden sein muß.

Erfindungsgemäß können sowohl zeitbezogene Temperaturwerte als auch zeitbezogene temperaturproportionale Werte verwendet werden. Sofern (vor allem in den Ansprüchen) von "ermittelten Zeit-/Temperaturwerten" die Rede ist, beziehen sich diese sowohl auf zeitbezogene Temperaturwerte als auch zeitbezogene temperaturproportionale Werte.

Als besonders vorteilhaft hat es sich erwiesen, zeitbezogene temperaturproportionale Werte zur Bildung der normierten Prüfwerte zu verwenden, da diese einfach berührungslos durch zum Beispiel Infrarot-Strahlungsmeßgeräte ermittelt werden können. Je nach Abstand zur Schmelze, den Übertragungseigenschaften der entsprechenden Sensoren und der Umgebungstemperatur liefern diese nur qualitative Werte, die aber stets temperaturproportional sind und durch die Normierung aussagekräftige Vergleichswerte liefern.

Bevorzugte Ausführungsbeispiele der Erfindung werden nachfolgend anhand der Zeichnung erläutert. In der Zeichnung zeigen:

Fig. 1 ein Diagramm mit einer Sollkurve und einer Meßkurve aus gemessenen Einzelwerten,

Fig. 2 das Diagramm gemäß der Fig. 1 mit einer zusätzlichen gestrichelt dargestellten normierten Meßkurve, und

Fig. 3 ein Vergleichsdiagramm aus zwei (oberen und unteren) Grenzkurven und der wiederum gestrichelt dargestellten normierten Meßkurve gemäß der Fig. 2.

Beim Gießen von Dentalgußteilen wird ein Rohling des zu gießenden Materials aus beispielsweise einer Titanlegierung geschmolzen und anschließend in eine entsprechende Gußform gegossen. Entscheidend für die Qualität des zu gießenden Dentalteils ist die Zusammensetzung der Gußlegierung. Diese ist daher erfindungsgemäß auf einfache und aussagekräftige Weise zu überprüfen und zu dokumentieren.

Beim im folgenden beschriebenen Verfahren wird während des Schmelzens des Gußmaterials, also zeitabhängig, die an der Oberfläche des zunächst noch festen Gußmaterials und später flüssigen Gußmaterials abgestrahlte Wärme gemessen. Vorzugsweise werden Infrarot-Strahlungswerte ermittelt. Diese Werte sind proportional zur momentanen Temperatur des Gußwerkstoffs. Mit steigender Temperatur nimmt somit auch die Infrarot-Strahlungsenergie zu. Diese so gewonnenen Meßwerte können deshalb qualitativ als Zeit-Temperaturwerte angesehen werden, weswegen sie im folgenden auch vereinfacht so genannt werden.

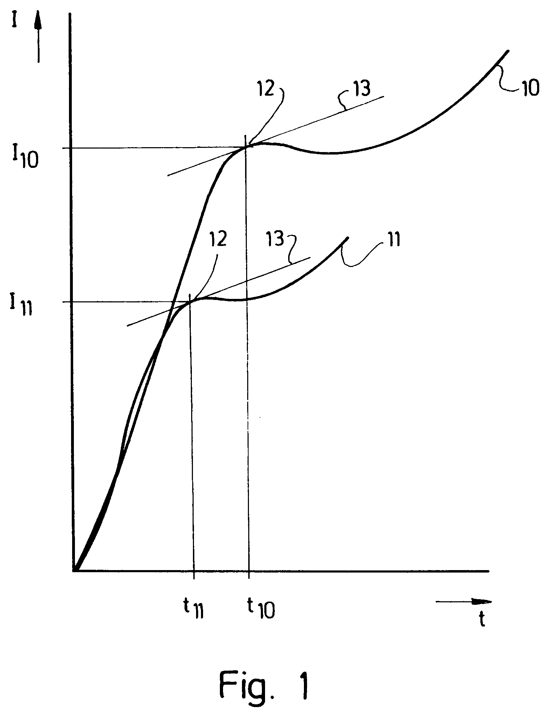

Zunächst werden von einer bestimmten, idealen Legierung während des Schmelzens zeitabhängige temperaturproportionale Sollwerte, nämlich zeitabhängige Strahlungswerte, ermittelt. Hieraus wird eine ein Vergleichsdiagramm darstellende Sollkurve 10 gebildet. Nachdem die Sollkurve 10 für die betreffende Legierung einmal ermittelt worden ist, dient sie als Vergleichskurve für alle zu prüfenden Legierungen, die der Legierung der Sollkurve 10 entsprechen sollen. Die anhand der jeweils zu prüfenden Legierung ermittelten zeitabhängigen Infrarot-Strahlungswerte werden zur Bildung einer Meßkurve 11 herangezogen. In der Fig. 1 sind sowohl die Sollkurve 10 als auch die Meßkurve 11 dargestellt. Letztere weicht von der Sollkurve 10 ab. Das kann verschiedene Gründe haben: Der Hauptgrund liegt darin, daß die Sollkurve 10 mit einer anderen Materialmenge aufgenommen worden ist als die Meßkurve 11. Ein weiterer Grund besteht darin, daß der Abstand des Meßsensors von der Oberfläche des zu messenden Materials größer oder kleiner ist als bei der Sollkurve 10. Um die von der Sollkurve 10 abweichende Meßkurve 11 zu Prüfzwecken mit der Sollkurve 10 vergleichen zu können, erfolgt erfindungsgemäß eine Normierung der Meßkurve 11.

Die Normierung der Meßkurve 11 erfolgt unter Zuhilfenahme eines charakteristischen Einzelpunkts. Vorzugsweise wird dazu ein Soliduspunkt 12 herangezogen. Der Soliduspunkt 12 liegt dort, wo das feste Gußmaterial anfängt, füssig zu werden. Zur Verflüssigung des festen Materials wird eine Aggregatzustandsänderungsenergie benötigt, die dazu führt, daß über einen gewissen Zeitpunkt hinweg sich die Temperatur und damit die Strahlungsenergie des Gußmaterials nicht oder nur noch unwesentlich ändert. Charakterisiert wird der Soliduspunkt 12 sowohl in der Sollkurve 10 als auch in der Meßkurve 11 durch eine Steigung der Kurve, die nahe 0 ist. Im Soliduspunkt 12 verläuft also die Kurve nahezu horizontal. Zeichnerisch läßt sich der Soliduspunkt 12 ermitteln durch eine leicht zur Horizontalen geneigte Tangente 13, mit der ein Schnittpunkt der Sollkurve 10 bzw. der Meßkurve 11 gebildet ist. Dort, wo die parallelen Tangenten 13 die Sollkurve 10 einerseits und die Meßkurve 11 andererseits schneiden, befindet sich der jeweilige Soliduspunkt 12 (Fig. 1). Rechnerisch läßt sich der Soliduspunkt 12 ermitteln durch die Differenz zweier in einem bestimmten, kurzen Zeitintervall aufeinanderfolgender Strahlungswerte. Verringert sich diese Differenz bis zu einem bestimmten vorgegebenen Differenzwert, ist das der gesuchte Soliduspunkt 12. Neben einer Differenzenbildung kann der Soliduspunkt 12 rechnerisch auch durch ein Differential, nämlich durch Ableitung der Strahlung nach der Zeit, ermittelt werden.

Zur Normierung der Meßkurve 11 werden zunächst die Strahlungswerte und die Zeitwerte am Soliduspunkt 12 der Sollkurve 10 zu den entsprechenden Werten am Soliduspunkt 12 der Meßkurve 11 ins Verhältnis gesetzt, und zwar nach folgenden Gleichungen:

$$I_{10} : I_{11} = F_I \quad t_{10} : t_{11} = F_t$$

Mit diesen Faktoren ($F_I$; $F_t$) werden die im Soliduspunkt 12 liegenden Emissions- und Zeitwerte der Meßkurve 11 multipliziert zum normierten Soliduspunkt, der dem Soliduspunkt 12 der Sollkurve 10 entspricht. Dazu werden folgende Rechnungen ausgeführt:

$$I_{11} \times F_I = I_{14} \quad t_{11} \times F_t = t_{14}$$

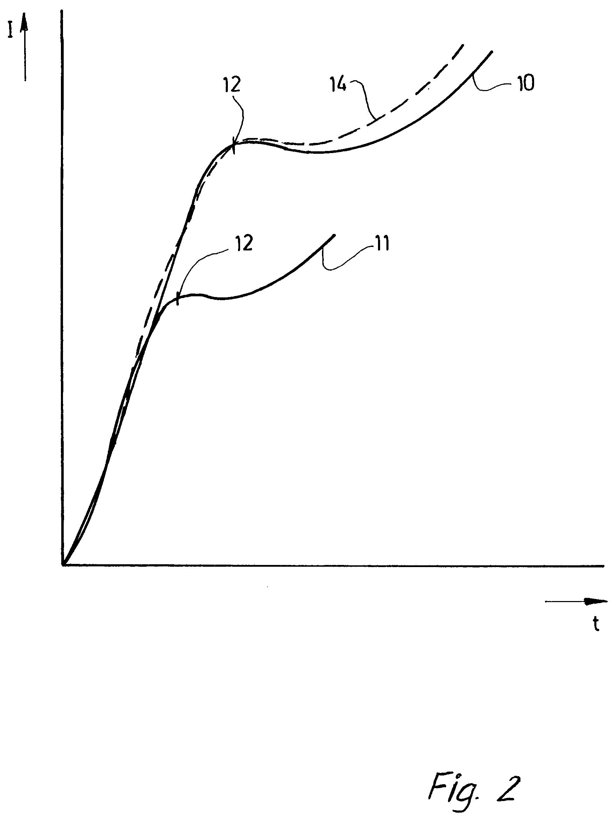

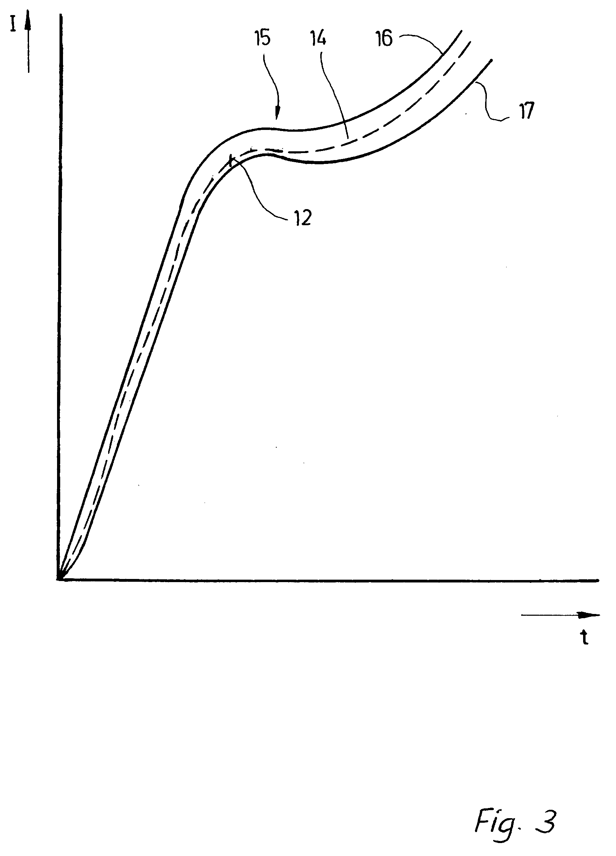

Die übrigen zeitabhängigen und temperaturproportionalen Strahlungswerte der Meßkurve 11 werden mit den gleichen Faktoren $F_I$ und $F_t$ multipliziert. Die sich dabei ergebenden normierten Werte führen zur normierten Meßkurve 14. Diese ist in den Fig. 2 und 3 gestrichelt dargestellt. In der Fig. 2 wird deutlich erkennbar, daß der Soliduspunkt 12 der Sollkurve 10 mit dem Soliduspunkt 12 der normierten Meßkurve 14 zusammenfällt.

Die normierte Meßkurve 14 bildet eine Prüfkurve, die mit der die Vergleichskurve darstellenden Sollkurve 10 vergleichbar ist. Beim visuellen Vergleich der Sollkurve 10 mit der normierten Meßkurve 14 erfolgt eine Überlagerung der beiden Kurven. Das kann dadurch geschehen, daß auf ein transparentes Schablonenblatt die Sollkurve 10 in einem Koordinatensystem aufgetragen wird. Ein Vergleich mit der normierten Meßkurve 14 erfolgt derart, daß auf die normierte Meßkurve 14 das transparente Blatt mit der Sollkurve 10 als Schablone bei sich deckenden Koordinatensystemen aufgelegt wird. Es ergeben sich dann die beiden Kurvenverläufe der in der Fig. 2 eingezeichneten Sollkurve 10 und der normierten Meßkurve 14. Soweit die Abweichungen wie im in der Fig. 2 gezeigten Beispiel nur sehr gering sind, kann davon ausgegangen werden, daß die mit der normierten Meßkurve 14 geprüfte Legierung der gewünschten Legierung im wesentlichen entspricht.

In der Fig. 3 findet ein Vergleichsdiagramm 15 Verwendung, das sich aus zwei Grenzkurven 16 und 17 zusammensetzt. Die Grenzkurven 16 und 17 sind gebildet aus der Sollkurve 10, indem die obere Grenzkurve 16 die Maximalwerte und die untere Grenzkurve 17 die Minimalwerte angibt. Im von den Grenzkurven 16 und 17 eingeschlossenen (streifenförmigen) Bereich, der sich zu zunehmenden Strahlungs- bzw. Zeitwerten hin leicht vergrößert, also breiter wird, muß die normierte Meßkurve 14 liegen, wenn die zu prüfende Legierung in Ordnung ist. Ein solches Beispiel ist in der Fig. 3 dargestellt, wo die normierte Meßkurve 14 den Bereich zwischen den Grenzkurven 16 und 17 nicht verläßt.

Die bei der Prüfung der zu schmelzenden Legierung gewonnenen Meßwerte, nämlich in regelmäßigen Zeitabständen aufgenommenen Infrarot-Emissionswerte, werden zweckmäßigerweise in einen Rechner eingegeben, der die Normierung, also die Umrechnung jedes Meßwerts mit dem Faktor $F_I$ und $F_t$, vornimmt. Somit ermittelt der Rechner die einzelnen Punkte der normierten Meßkurve 14. Diese Punkte werden als normierte Meßkurve 14 ausgegeben durch beispielsweise einen an die Schmelzvorrichtung oder eine Gußvorrichtung angeschlossenen Drucker. Dadurch entsteht ein Meßprotokoll, dessen normierte Meßkurve 14 mit einer auf einer Schablone aufgezeichneten Sollkurve 10 oder auf einer Schablone aufgezeichneten Grenzkurven 16 und 17 visuell verglichen wird. Alternativ ist es aber auch denkbar, durch den Drucker mit der normierten Meßkurve 14 gleich die Sollkurve 10 bzw. die Grenzkurven 16 und 17 auszudrucken. Dann ist ohne die Zuhilfenahme einer Schablone eine sofortige Auswertung möglich, und zwar jederzeit. Dadurch wird das Prüfergebnis dauerhaft dokumentiert.

Alternativ ist es auch möglich, auf einem Rechnerbildschirm die Sollkurve 10 bzw. die Grenzkurven 16, 17 und die normierte Meßkurve 14 darzustellen. Dabei wird die normierte Meßkurve 14 mit fortschreitender Erwärmung der Gußlegierung vervollständigt, wobei man gegebenenfalls den Schmelzprozeß vorzeitig abbrechen kann, wenn sich herausstellt, daß die normierte Meßkurve 14 über den erlaubten Bereich hinaus von der Sollkurve 10 abweicht.

Das vorstehend beschriebene Verfahren ist darauf beschränkt, die Prüfung anhand der zeitbezogenen Strahlungsenergie des Gußmaterials durchzuführen. Es können auch andere temperaturproportionale Werte oder tatsächliche Temperaturwerte herangezogen werden.

**Patentansprüche**

1. Verfahren zum Prüfen von Dentalgußmaterialien, bei dem zeitabhängige Temperaturwerte oder zeitabhängige temperaturbezogene Werte (Zeit-/Temperaturwerte) während des Schmelzens des Dentalgußmaterials ermittelt werden, **dadurch gekennzeichnet,** daß mindestens ein ermittelter Zeit-/Temperaturwert auf einen bestimmten Sollwert normiert wird und wenigstens dieser normierte Zeit-/Temperaturwert mit mindestens einem Sollwert verglichen wird.

2. Verfahren nach Anspruch 1, dadurch gekennzeichnet, daß die Normierung auf einen bestimmten, vorzugsweise charakteristischen, Zeit-/Temperatursollwert, insbesondere den Soliduspunkt (12), erfolgt.

3. Verfahren nach Anspruch 2, dadurch gekennzeichnet, daß als Soliduspunkt (12) die zeitbezogene Solidustemperatur oder die zeitbezogene solidusproportionale Temperatur verwendet wird.

4. Verfahren nach Anspruch 2 oder 3, dadurch gekennzeichnet, daß der Soliduspunkt (12) in Abhängigkeit von einem festgelegten Temperaturanstieg oder einer Temperaturkonstanz während eines bestimmten, insbesondere kurzen, Zeitintervalls ermittelt wird.

5. Verfahren nach einem oder mehreren der Ansprüche 1 bis 4, dadurch gekennzeichnet, daß die Normierung rechnerisch bzw. rechnerunterstützt erfolgt, insbesondere derart, daß der anhand der ermittelten Zeit-/Temperaturwerte festgestellte Soliduspunkt (12) hinsichtlich der Zeitwerte und/oder der Temperaturwerte bzw. der temperaturproportionalen Werte mit dem aus den Zeit-/Temperatursollwerten ermittelten Soliduspunkt zur Bildung von Verhältnisfaktoren ins Verhältnis gesetzt wird und mit den so gewonnenen Verhältnisfaktoren die ermittelten Zeit-/Temperaturwerte umgerechnet, insbesondere multipliziert, werden.

6. Verfahren nach einem oder mehreren der Ansprüche 1 bis 5, dadurch gekennzeichnet, daß die normierten Zeit-/Temperaturwerte mit den Zeit-/Temperatursollwerten derart, vorzugsweise rechnerisch oder visuell , verglichen werden, daß überprüft wird, ob die normierten Zeit-/Temperaturwerte den jeweiligen Zeit-/Temperatursollwert mehr als um einen bestimmten Wert über- bzw. unterschreiten.

7. Verfahren nach einem oder mehreren der Ansprüche 1 bis 6, dadurch gekennzeichnet, daß ein Vergleichsdiagramm (15) oder eine Sollkurve (10) mit den Zeit-/Temperatursollwerten einer bestimmten Legierung des Dentalgußmaterials verwendet wird.

8. Verfahren nach einem oder mehreren der Ansprüche 1 bis 7, dadurch gekennzeichnet, daß mit dem Vergleichsdiagramm (15) oder der Sollkurve (10) eine normierte Meßkurve (14) mit normierten, ermittelten Zeit-/Temperaturwerten eines zu prüfenden Dentalgußmaterials gleicher oder vergleichbarer Legierung, insbesondere eine normierte IR-Emissionskurve, überprüft wird.

9. Verfahren nach einem oder mehreren der Ansprüche 1 bis 8, dadurch gekennzeichnet, daß die normierte Meßkurve (14) und gegebenenfalls (gleichzeitig) das Vergleichsdiagramm (15) oder die Sollkurve (10) von einem der Schmelz- oder Gießvorrichtung zugeordneten Drucker oder dergleichen ausgedruckt werden.

10. Verfahren nach einem oder mehreren der Ansprüche 1 bis 9, dadurch gekennzeichnet, daß das Vergleichsdiagramm (15) zwei obere und untere Grenzwerte der Zeit-/Temperatursollwerte darstellende Grenzkurven (16, 17) aufweist, anhand derer überprüft wird, ob die normierte Meßkurve (14) in der von den Grenzkurven (16, 17) eingeschlossenen Fläche liegt.

11. Verfahren nach einem oder mehreren der Ansprüche 1 bis 10, dadurch gekennzeichnet, daß als Zeit-/Temperaturwerte temperaturproportionale Strahlungswerte des Dentalgußmaterials verwendet werden, die insbesondere mit einem IR-Strahlungsmeßgerät ermittelt werden.

Fig. 1

Fig. 2

Fig. 3